Europäisches Patentamt

European Patent Office

Office européen des brevets

(11) Publication number: **0 143 761**
**B1**

# (12) EUROPEAN PATENT SPECIFICATION

(45) Date of publication of patent specification: **16.03.88**

(51) Int. Cl.⁴: **A 61 B 5/04**

(21) Application number: **84850320.7**

(22) Date of filing: **24.10.84**

(54) **Electrode, fixed and stabilised by vacuum.**

(30) Priority: **28.10.83 SE 8305947**

(43) Date of publication of application:
**05.06.85 Bulletin 85/23**

(45) Publication of the grant of the patent:
**16.03.88 Bulletin 88/11**

(84) Designated Contracting States:
**AT BE CH DE FR IT LI LU NL SE**

(56) References cited:
**CH-A- 301 243**
**DE-A-2 064 781**
**DE-A-2 152 808**
**FR-A-1 139 191**
**FR-A-1 535 432**
**FR-A-2 155 247**
**SU-A- 171 966**

(73) Proprietor: **Astra-Tech Aktiebolag**
**Arstaängsvägen 1A**
**S-117 43 Stockholm (SE)**

(72) Inventor: **Lundbäck, Stig**
**Östra Tynningö**
**S-185 00 Vaxholm (SE)**

(74) Representative: **Hjertman, Ivan T. et al**
**AB ASTRA Patent and Trade Mark Department**
**S-151 85 Södertälje (SE)**

Courier Press, Leamington Spa, England.

## Description

The invention relates to an electrode, and more particularly to an electrode for use when making electrocardiograms (ECG), of the kind defined in the preamble of Claim 1.

Such an electrode is known from Austrian Patent Specification No. 248 608. This known electrode is attached through the agency of a vacuum applied through a tube connected to the electrode. A valve which assists in the application of the vacuum is held closed when the electrode is not attached, opens automatically when applying the electrode, and closes automatically should the electrode fall off. According to one described embodiment, an electrode plate is spring-biassed with the aid of an elastic diaphragm coupled to a surrounding sealing ring. When the electrode plate is pressed against the skin of a patient, a valve opens and a vacuum is applied to a cavity extending around the electrode and defined by the sealing ring.

The elastic diaphragm permits the electrode plate to swivel and to move axially in relation to the sealing ring. Unfortunately, it has been found that because of this freedom of movement the electrode tends to fall away, unless very low vacuum-pressures are used, i.e. pressures which leave pronounced suction marks. Moreover, there is no guarantee that the electrode pressure will remain constant, and hence the electrical function of the electrode is dependent upon the patient lying quite still. Very small variations are sufficient to produce changes in the contact resistance, such as to cause the base line against which the ECG-variable is reproduced on a writer or like recording instrument to become unsteady and variable. Consequently, this method of attaching electrodes to a patient by vacuum through the agency of centrally located suction means, a method which is both practical and advantageous in theory with respect to many electrodes, has not found wide use in practice.

An object of the present invention is to provide an electrode of the kind described in the introduction which will remain firmly seated, therewith to better fulfil its intended purpose; which will remain firmly attached to a patient even though the patient should move during the examination; which will avoid the occurrence of base-line variations due to changes in surface-contact pressure; and which will enable functional tests to be made on the heart of a patient during movement of the patient, something which from a diagnostic aspect is highly desirable, but which is so difficult to carry out when using conventional techniques, as to make it impossible to include such a feature in general routine procedures.

These objects and associated advantages are realized in accordance with the invention by means of an electrode having the characterizing features set forth in the characterizing claus of Claim 1. Advantageous embodiments are defined in the depending claims.

In accordance with the invention there is provided a suction-held electrode which exhibits a relatively rigid connection between an electrode plate and a surrounding sealing ring provided with a sealing lip. The sealing lip is preferably slightly pliable, but not excessively so, such that sealing is effected via a moderately curved surface.

An electrode which is held in position by means of suction forces and which has a surrounding sealing ring which is relatively rigidly connected to a centrally located electrode plate is known from the US Patent Specification No. 4,248,242. With this electrode, however, the suction forces cannot be applied through a tube from a central vacuum source, since this electrode design lacks the self-closing valve of the Austrian patent, which valve cannot be combined with the rigid electrode-structure. Instead, this known electrode is supplied with pressurized air, which drives an ejector-suction device incorporated in the electrode itself. This causes the electrode to be highly disturbing, especially to a patient undergoing an examination, since it emits a high whining noise when seated on the body of the patient. In addition hereto, everything that enters the ejector pipe of the suction device is blown therethrough into the room, for example aerosols containing electrode-paste applied to the electrode, and non-sterile body fluid (from transpiration), and hence when using this electrode there is obtained both sound and other apparent sanitary inconveniences. Because of its structural design, such an electrode cannot be sterilized, and neither can it be used in the same manner as a disposable electrode. In addition, the air which drives the ejector must be at a relatively high pressure, approximately 0.6 kg/cm$^2$, which results in sealing problems and requires the use of pressure hoses.

At present there are used mainly two types of electrodes. Firstly, there is used the disposable-type electrode, which is attached with the aid of an adhesive (glue, adhesive tape etc.) or with the aid of rubber bands or the like. Secondly there is used the multi-use electrode which is held in position by a locally generated vacuum force. One well known vacuum principle employs the compression and subsequent expansion of rubber balls. Unfortunately, due to insufficient reservoir sizes, these balls loosen in the event of even very slight leaks, which leads to serious problems when wishing to work, for example, with six electrodes affixed to the patient at the same time, which is normal routine procedure. Consequently, none of these systems is fully satisfactory. These disadvantages are eliminated to a great extent by means of the invention.

According to one advantageous aspect of the invention, the electrode provided thereby may partially have the form of a disposable-type electrode or may comprise readily exchangable, sterilizable components. Suitably, the actual electrode plate has the form of a small, throw-away "button", since for electrochemical reasons the best material from which the electrode surface can be produced is silver, with a coating of silver chloride, this material being blackened by light. In this respect, a metal or plastic electrode provided with press-stud attachment means can be given a very thin layer of silver. The sealing ring can also be made of an elastomeric material and the rings changed upon being used once, and collected, washed and sterilized for re-use, unless wishing to design the rings for one-time use only.

The invention will now be described with reference to an exemplifying embodiment thereof, illustrated in the accompanying drawing. Figure 1 is an exploded view of an electrode which comprises three parts. Figure 2 is a sectional view of an electrode in an inoperative position, with the vacuum valve closed. Figure 3 is a view of an electrode when affixed to a patient.

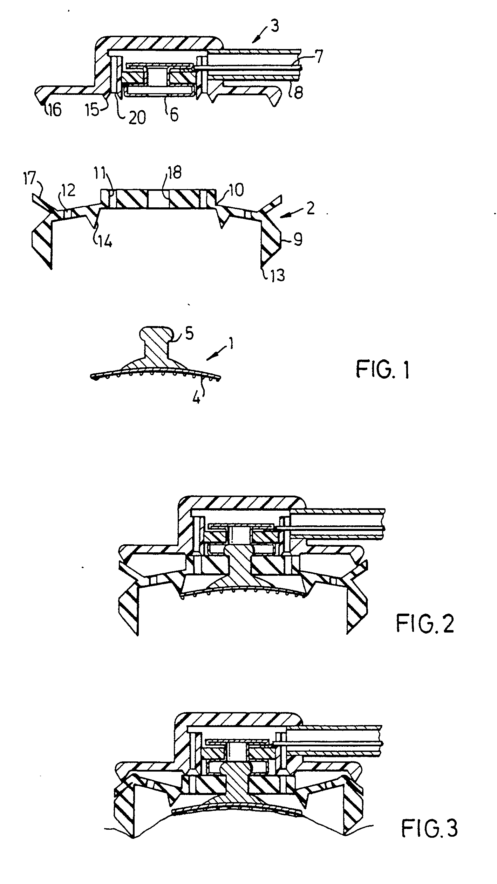

The various components of the illustrated embodiment are best seen from Figure 1. An electrode plate 1, which may be made of metal or of a plastics material having a metal surface 4, is provided with protruding portions to afford good contact with the skin, even in the presence of body-hair. Located on the rear side of the plate is a stem 5, which forms the male component of a press-stud connector. The stem 5 is intended to be pushed into a press-stud female part 6 located in a backpiece 3, therewith penetrating a hole 18 in an intermediate plate or insert 2, which incorporates, inter alia, a sealing ring 9. As will be understood, the press-stud connector is of a conventional kind used with jeans. The backpiece 3 may comprise an electrically non-conductive plastics material, optionally provided with a metallic screening means (not shown). Connected to the backpiece is a vacuum hose or pipe 8, through which a screened conductor 7 is drawn to the metallic press-stud connector 6. Thus, when the connector parts 5, 6 are pressed together, the electrode plate is firmly attached to the backpiece 3 and is connected through the electrical conductor 7 to a conventional current-supply source. The vacuum hose is connected to a cavity in the backpiece 3 and a plurality of holes 20 are located around female part 6 of the connector.

Figure 2 illustrates the electrode components of Figure 1 in their assembled state. For the sake of clarity, only Figure 1 has been provided with component-identifying references. However, the components illustrated in the remaining Figures can be readily identified, by simple comparison with Figure 1. The components 1 and 2 of the illustrated embodiment are rotationally symmetrical, thereby facilitating their manufacture. Such symmetry is not absolutely necessary, however.

The insert 2, which is preferably made of silicone rubber, is provided with a relatively rigidly formed ring portion 9 having extending peripherally therearound a sealing lip 13, which seals against the skin of the in-use or operative position. The mode of the electrode in the inoperative state thereof will be described first, with reference to Figure 2.

It will be seen that the relatively flat centre part of the insert, exhibiting hole 18, will lie against the sealing lip 15 located on the backpiece 3. The upper side of this centre part, as seen in Figure 1, communicates with the lower side thereof through circumferentially located holes 11. Located on this lower side of the centre part is a further sealing lip 14. The lip 14 abuts the rear side of the electrode plate 1, and both the lip 14 and the lip 15 seal-off a cavity to which a vacuum is applied and which is defined by said centre part and the electrode plate, therewith a certain amount of deflection is obtained in said centre part of the insert 2, which contributes towards resilient abutment of a flange 17, located on the insert 2, with a further lip 16 on the backpiece 3. Thus, in the configuration illustrated in Figure 2, apart from insignificant leakages, only the aforementioned cavity behind the electrode plate 1 will be placed under a vacuum.

When a vacuum is applied to the electrode assembly shown in Figure 2 and the electrode is placed on the skin of a patient (optionally after applying an electrode paste, etc., which can be omitted, however), the following happenings occur. When the circumferentially extending lip 13 is pressed against the skin, the force applied herewith will act upon the flange 17, via the relatively inflexible ring 9, therewith to resiliently deform the flange, whereupon the centre part of the insert 2 is deformed and the lip 14 eases away from the rear side of the electrode plate 1. The space or cavity between the skin and the electrode plate 1 is placed in communication with the vacuum source, and since the insert 2 is provided with holes 12 all spaces or electrode-cavities will be placed under vacuum, the sealing lips 13 and 16 therewith sealing between the backpiece 3 and the skin. The configuration illustrated in Figure 3 is then reached.

It will be clear from Figure 3 that the backpiece 3 and the ring 9 now function as an interlocking composite assembly. Although the ring 9 is urged outwardly, away from the backpiece 3 by an elastic deformation force acting through the flange 17, this force is quite insignificant in comparison with the pneumatic forces. The vacuum used need not reach more than $0.1 \text{ kg/cm}^2$. The air pressure then exerts against the skin a force which corresponds substantially to the force exterted by the surface embraced by the lip 13. This force is counter-acted by the resistance normally offered by the skin, the greater part of which resistance is exerted on the undersurface 4 of the electrode plate 1, thereby to achieve particularly good contact. Due to deformation of the skin, there is also obtained a certain shape-conforming effect, or imbedding effect, which prevents the electrode plate from slipping. Electrode-paste and transpiration tend to reduce friction, and hence this imbedding of the electrode is necessary in order to hold the electrode firm against the action of break forces and shear forces. In reality, a break force applied via the hose 8 will cause the skin to accompany the movement until deformation is too great and the lip 13 is no longer able to provide a seal, whereupon the electrode will, of course, fall off, and the resilient flange 13 return to the position shown in Figure 2, with the valve seal once again in effect.

Thus, in order to obtain good functioning, the electrode surface 4 in the position illustrated in Figure 3 must be inwardly drawn relative to the lip 13 on the ring 9. With a lip of diameter 30 mm, the electrode surface is suitably inwardly drawn to a depth of 3—4 mm (In the Figures, the illustrated electrode assembly is enlarged by a factor of two).

The described embodiment has been found to function extremely well in practice. For example, six electrodes can be affixed to a patient very quickly, since no taps need be opened and since all that is required is for the electrodes to be simply pressed, one at a time, onto the skin of the patient, the position where each electrode is to be applied suitably being identified by appropriate symbols placed on the rear surface of respective backpieces. It is found that the presence of body hair presents no problems in this respect, and that the electrodes will remain firmly in position, even should the patient jump barefooted onto the floor. Because of the low suction pressure applied, a tenth of an atmosphere is sufficient, the skin of a normal person will not be marked by the suction applied thereto, apart from a faint red ring where the lip 13 has lain, even should the electrodes be left attached for thirty minutes. Subsequent to using the electrode and disconnecting the vacuum source, all that need be done is to strip off the electrode plate 1 via the press-stud connector, wherewith the elastomeric insert 2 will follow suit, and to place new, or sterilized components on the backpiece 3, whereupon the electrode is ready for use with a further patient. The components can be readily sterilized, and large numbers can be sterilized at the same time. Thus, electrodes designed in accordance with the invention are extremely practical and hygienic, and permit examinations which have hitherto been troublesome, due to deficiences in electrode functions, to be carried out as a matter of routine. Because of the electrical reliability and stability of the electrode contact, these examinations can be carried out more quickly than was possible hitherto, and previously applied prolonged sampling methods can be shortened. In certain cases, particularly when the electrode is to be used for a longer period of time or for examinations where patients are placed under body stress, it may be suitable to incorporate in the electrode some form of liquid absorbing material, for example blotting paper or the like, this material being placed between the elastomeric component and the backpiece, and being discarded after use.

**Claims**

1. An electrode particularly intended for electrocardiogram examinations and the like, and which is intended to be attached with the aid of a hose or tube through which a vacuum is applied from a vacuum source, and which electrode comprises an electrically conductive electrode plate (1) which can be coupled to a measuring instrument (via 7), and which lies against a surface of, for example, skin via an electrode surface (4), and a sealing means (2) which surrounds the electrode plate (1) and includes a sealing ring (9) which extends circumferentially around the electrode plate and terminates in a sealing lip (13) and which, when the electrode occupies its in-use position, abuts said surface, such that in said in-use position said surface and said sealing means define together a cavity to which a vacuum is applied from said vacuum source, said electrode plate and said sealing lip (13) being movable relative to one another, under the action of a resilient force (via 17) which strives to separate the electrode plate from a plane defined by said sealing lip, from a working position (Figure 3) to a rest position (Figure 2), said movement being arranged to activate closure of a valve means, to shut-off the vacuum-supply to said cavity under vacuum, characterized in that it further comprises an inflexible backpiece (3) supporting the electrode plate (1), in that the electrode plate, in both the working position and the rest position, adopts the same positions relative to the backpiece (3); and in that the sealing lip (13) is seated on a movable ring (9), which is pressed against the backpiece (3) in said working position.

2. An electrode according to Claim 1, characterized in that both the electrode plate (1) and the sealing means (2) can be removed from the backpiece (3) and exchanged.

3. An electrode according to Claim 1, characterized in that the sealing means comprises an elastomeric single-piece component (2) which includes said sealing ring (9) incorporating the sealing lip (13); in that said sealing ring on the part thereof remote from the sealing lip (13) exhibits a diaphragm having located centrally therein a hole (18) through which a stem part (5) of the electrode is intended to pass; and in that the backpiece (3) is provided with holding means (6) for co-action with said stem part (5).

4. An electrode according to Claim 3, characterized in that the elastomeric component is provided with resilient means (17) compressible into resilient abutment with said backpiece (3); in that the centre part of said elastomeric component co-acts with the backpiece via first sealing means (15), inwardly of which the elastomeric component has at least one through-passing opening (11) for producing a vacuum on the front side thereof; and in that outwardly of said at least one through-passing opening (11) there is arranged a second sealing means (14) for effecting a seal against the upper side of the electrode plate in the relaxed uncompressed state of the resilient means (17) and the sealing effect of which is arranged to cease when the sealing ring (9) is moved towards the backpiece against the action of said resilient means (17).

5. An electrode according to Claim 4, characterized in that the elastomeric component has located between the sealing ring (9) and said further sealing means (14) at least one through-passing opening (12); and in that said resilient means (17) comprises a circumferentially extending flange (17) which sealingly abuts a sealing lip (16) on the backpiece (3), whereby in the active position all electrode cavities are in communication with said vacuum source.

6. An electrode according to Claim 1, characterized in that in the active position of the electrode the electrode plate occupies a withdrawn position relative to said sealing lip (13).

7. An electrode according to Claim 1, characterized in that the electrode surface (4) of the electrode plate is concave.

8. An electrode according to Claim 7, characterized in that the electrode surface (4) of the electrode plate is provided with small protruding portions.

9. An electrode according to Claim 3, characterized in that the means for detachably holding the electrode plate to the backpiece comprises a press-stud mechanism.

10. An electrode according to any one of the preceding Claims, characterized in that coupling of the electrode to a measuring instrument is effected via an electrical, preferably screened conductor (7) which is drawn through a vacuum tube or hose (8) connected to said backpiece.

11. An electrode plate for use with an electrode according to any one of Claims 1 to 6, characterized in that said electrode plate has a front surface (4) comprising an electrode surface coated with a layer of silver chloride, the rear side of the electrode plate being provided with a stem (5) with a thickened portion at one end thereof and serving as the male component of a press-stud connector.

12. An electrode plate according to Claim 11, characterized in that the front surface (4) has protruding portions arranged thereon (Figure 1).

**Patentansprüche**

1. Elektrode, insbesondere für Elektrokardiogrammuntersuchungen u.dgl., welche mit Hilfe eines Schlauches oder Rohres angebracht werden soll, durch welchen bzw. welches ein Vakuum von einer Vakuumquelle angelegt wird und welche Elektrode umfaßt: eine elektrisch leitfähige Elektrodenplatte (1), die mit einem Meßinstrument (über 7) gekoppelt werden kann und die über eine Elektrodenoberfläche (4) gegen eine Oberfläche, beispielsweise Haut, anliegt, und ein Dichtungsmittel (2), das die Elektrodenplatte (1) umgibt und einen Dichtungsring (9) inkludiert, welcher sich in Umfangsrichtung um die Elektrodenplatte herum erstreckt und in einem Dichtungsansatz (13) endigt, und welches, wenn die Elektrode sich in ihrer Gebrauchsposition befindet, gegen diese Oberfläche so anliegt, daß in dieser Gebrauchsposition die Oberfläche und das Dichtungsmittel miteinander einem Hohlraum begrenzen, an welchem ein Vakuum von der Vakuumquelle angelegt wird, wobei die Elektrodenplatte und der Dichtungsansatz (13) in bezug zueinander unter der Wirkung einer Federkraft (über 17), die danach strebt, die Elektrodenplatte von einer vom Dichtungsansatz festgelegten Ebene zu entfernen, von einer Arbeitsposition (Fig. 3) in einer Ruheposition (Fig. 2) beweglich sind, wobei diese Bewegung so beschaffen ist, daß der Verschluß eines Ventilmittels aktiviert wird, um die Vakuumzufuhr zu dem unter Vakuum befindlichen Hohlraum abzuschalten, dadurch gekennzeichnet, daß sie weiters ein unbiegsames Stützelement (3) umfaßt, welches die Elektrodenplatte (1) stützt; daß die Elektrodenplatte, sowohl in der Arbeitsposition als auch in der Ruheposition, dieselbe Stellung in bezug auf das Stützelement (3) einnimmt; und daß der Dichtungsansatz (13) auf einen beweglichen Ring (9) angeordnet ist, welcher in der Arbeitsposition gegen das Stützelement (3) gepreßt ist.

2. Elektrode nach Anspruch 1, dadurch gekennzeichnet, daß sowohl die Eletrodenplatte (1) als auch das Dichtungsmittel (2) vom Stützelement (3) entfernt und ausgetauscht werden können.

3. Elektrode nach Anspruch 1, dadurch gekennzeichnet, daß das Dichtungsmittel einen elastomeren einstückigen Bestandteil (2) umfaßt, welcher den Dichtungsring (9) mit inkorporiertem Dichtungsansatz (13) inkludiert; daß der Dichtungsring an dem vom Dichtungsansatz (13) entfernten Teil ein Diaphragma mit einem Loch (18) in der Mitte desselben aufweist, durch welches ein Stielteil (5) der Elektrode hindurchtreten soll; und daß das Stützelement (3) mit einem Haltemittel (6) zum Zusammenwirken mit dem Stielteil (5) versehen ist.

4. Elektrode nach Anspruch 3, dadurch gekennzeichnet, daß der elastomere Bestandteil mit Federmitteln (17) versehen ist, welche in federnde Anlage an das Stützelement (3) preßbar sind; daß der Mittelteil des elastomeren Bestandteiles mit dem Stützelement über erste Dichtungsmittel (15) zusammenwirkt, innerhalb welcher der elastomere Bestandteil zumindest eine durchgehende Öffnung (11) zur Erzeugung eines Vakuums an der Vorderseite desselben aufweist; und daß außerhalb der zumindest einen durchgehenden Öffnung (11) ein zweites Dichtungsmittel (14) zum Bewirken einer Dichtung gegen die Oberseite der Elektrodenplatte im entspannten, nicht komprimierten Zustand der Federmittel (17) vorgesehen ist und dessen Dichtungswirkung so beschaffen ist, daß sie aufhört, wenn der Dichtungsring (9) gegen die Wirkung der Federmittel (17) zum Stützelement hin bewegt wird.

5. Elektrode nach Anspruch 4, dadurch gekennzeichnet, daß der elastomere Bestandteil zwischen dem Dichtungsring (9) und dem weiteren Dichtungsmittel (14) zumindest eine durchgehende Öffnung (12) aufweist; und daß die Federmittel (17) einen sich um den Umfang erstreckenden Flansch (17) umfassen, welcher dichtend an einen Dichtungsansatz (16) am Stützelement (3) anliegt, wodurch in der aktiven Position alle Elektrodenhohlräume in Verbindung mit der Vakuumquelle stehen.

6. Elektrode nach Anspruch 1, dadurch gekennzeichnet, daß in der aktiven Position der Elektrode die Elektrodenplatte in bezug auf den Dichtungsansatz (13) eine zurückgezogene Position einnimmt.

7. Elektrode nach Anspruch 1, dadurch gekennzeichnet, daß die Elektrodenoberfläche (4) der Elektrodenplatte konkav ist.

8. Elektrode nach Anspruch 7, dadurch gekennzeichnet, daß die Elektrodenoberfläche (4) der Elektrodenplatte mit kleinen vorstehenden Teilen versehen ist.

9. Elektrode nach Anspruch 3, dadurch gekennzeichnet, daß das Mittel zum lösbaren Halten der Elektrodenplatte am Stützelement einen Druckknopfmechanismus umfaßt.

10. Elektrode nach irgendeinem der vorhergehenden Ansprüche, dadurch gekennzeichnet, daß die Kopplung der Elektrode mit einem Meßinstrument über einen elektrischen, vorzugsweise abgeschirmten Leiter (7) bewirkt wird, der durch ein mit dem Stützelement verbundeness Vakuumrohr oder einem Vakuumschlauch (8) gezogen ist.

11. Elektrodenplatte zur Verwendung mit einer Elektrode gemäß irgendeinem der Ansprüche 1 bis 6, dadurch gekennzeichnet, daß die Elektrodenplatte eine Vorderfläche (4) aufweist, die eine mit einer Silberchloridschicht überzogene Elektrodenoberfläche umfaßt, wobei die Rückseite der Elektrodenplatte mit einem als männlicher Bestandteil einer Druckknopfverbindung dienenden Stiel (5) mit einem verdickten Abschnitt an einem Ende desselben versehen ist.

12. Elektrodenplatte nach Anspruch 11, dadurch gekennzeichnet, daß die Vorderfläche (4) an ihr angeordnete vorstehende Teile aufweist (Fig. 1).

**Revendications**

1. Electrode prévue notamment pour des examens d'électrocardiographie et analogues et qui est destinée à être fixée à l'aide d'un tuyau ou d'un tube, au moyen duquel un vide est appliqué à partir d'une source de vide, laquelle électrode comportant une plaque d'électrode électriquement conductrice (1), qui peut être accouplée à un instrument de mesure (par l'intermédiaire de 7) et s'applique contre une surface de la peau par exemple par l'intermédiaire d'une surface d'électrode (4), et un dispositif d'étanchéite (2) qui entoure la plaque d'électrode (1) et comporte une bague d'étanchéité (9) qui s'étend circonférentiellement autour de la plaque d'électrode et se termine par une lèvre d'étanchéité (13) et qui, lorsque l'électrode est dans sa position d'utilisation, est un butée contre ladite surface de sorte que, dans ladite position d'utilisation, ladite surface et ledit dispositif d'étanchéité définissent ensemble une cavité à laquelle un vide est appliqué à partir de ladite source de vide, ladite plaque d'électrode et ladite lèvre d'étanchéité (13) étant déplaçables l'une par rapport à l'autre, sous l'action d'une force élastique (par l'intermédiaire de 17), qui tend à séparer la plaque d'électrode d'un plan défini par ladite lèvre d'étanchéité, depuis une position de travail (figure 3) dans une position de repos (figure 2), ledit déplacement étant conçu de manière à déclencher la fermeture de moyens formant valve de manière à interrompre l'établissement du vide dans ladite cavité sous vide, caractérisée en ce qu'elle comporte en outre une pièce de soutien flexible (3) supportant la plaque d'électrode (1), en ce que la plaque d'électrode prend à la fois dans la position de travail et dans la position de repos, les mêmes positions par rapport à la pièce de soutien (3); et en ce que la lèvre d'étanchéité (13) est en appui sur une bague mobile (9) qui est repoussée contre la pièce de soutien (3) dans ladite position de travail.

2. Electrode selon la revendication 1, caractérisée en ce qu'à la fois la plaque d'électrode (1) et les moyens d'étanchéité (2) peuvent être retirés de la pièce de support (3) et être remplacés.

3. Electrode selon la revendication 1, caractérisée en ce que le dispositif d'étanchéité comporte un composant monobloc élastomère (2) qui comprend ladite bague d'étanchéité (9) comportant la lèvre d'étanchéité (13); en ce que ladite bague d'étanchéité comporte, sur sa partie distante de la lèvre d'étanchéité (13), un diaphragme au centre duquel se trouve ménagé un trou (18), qui est prévu pour être traversé par une partie formant tige (5) de l'électrode; et en ce que la pièce de soutien (3) comporte des moyens de retenue (6) destinés à coopérer avec ladite formant tige (5).

4. Electrode selon la revendication 3, caractérisée en ce que le composant élastomère est muni de moyens élastiques (17) pouvant être comprimés en étant appliqués élastiquement en butée contre ladite pièce de soutien (3); en ce que la partie centrale dudit composant élastomère coopère avec la pièce de soutien par l'intermédiaire du premier dispositif d'étanchéité (15), sur le côté intérieur duquel le composant élastomère comporte au moins une ouverture percée de part en part (11) servant à établir un vide sur la face avant du dispositif d'étanchéité; et en ce qu'à l'extérieur de ladite ouverture percée de part en part (11) se trouve disposé un second dispositif d'étanchéité (14) servant à établir une étanchéité contre la face supérieure de la plaque d'électrode lorsque les moyens élastiques (17) sont à l'état détendu, non comprimé, l'action d'étanchéité de ce second dispositif d'étanchéité étant conçue de manière à cesser lorsque la bague d'étanchéité (9) est déplacée en direction de la pièce de soutien à l'encontre de l'action desdits moyens élastiques (17).

5. Electrode selon la revendication 4, caractérisée en ce que le composant élastomère comporte au moins une ouverture percée de part en part (12) disposée entre la bague d'étanchéité (9) et ledit second dispositif d'étanchéité (14); et en ce que lesdits moyens élastiques (17) comportent une bride circonférentielle (17) qui est appliquée de façon étanche contre une lèvre d'étanchéité (16) située sur la pièce de soutien (3), ce qui a pour effet que, dans la position active, toutes les cavités de l'électrode sont en communication avec ladite source de vide.

6. Electrode selon la revendication 1, caractérisée en ce que, lorsque l'électrode est dans la position active, la plaque d'électrode occupe une position rétractée par rapport à ladite lèvre d'étanchéité (13).

7. Electrode selon la revendication 1, caractérisée en ce que la surface (4) de la plaque d'électrode est concave.

8. Electrode selon la revendication 7, caractérisée en ce que la surface (4) de la plaque d'électrode comporte de petites parties saillantes.

9. Electrode selon la revendication 3, caractérisée en ce que les moyens permettant de retenir de façon détachable la plaque d'électrode à la pièce de soutien comporte un mécanisme à broche de pression.

10. Electrode selon l'une quelconque des revendications précédentes, caractérisée en ce que l'accouplement de l'électrode à un instrument de mesure est réalisé par l'intermédiaire d'un conducteur électrique (7) de préférence blindé, qui s'étend à travers un tube ou un tuyau (8) d'établissement du vide, raccordé à la pièce de soutien.

11. Plaque d'électrode destinée à être utilisée avec une électrode selon l'une quelconque des revendications 1 à 6, caractérisée en ce que ladite plaque d'électrode possède une surface frontale (4) comportant une surface d'électrode recouverte par une couche de chlorure d'argent, la face arrière de la plaque d'électrode étant équipée d'une tige (5) munie d'une partie épaissie au niveau de l'une de ses extrémités et constituant le composant mâle d'un raccord à broche de pression.

12. Plaque d'électrode selon la revendication 1, caractérisée en ce que la surface frontale (4) comporte des parties saillantes disposées sur elle-même (figure 1).

0 143 761

FIG. 1

FIG.2

FIG.3

1